# EUROPÄISCHE PATENTSCHRIFT

(11) **EP 2 729 797 B1**
(45) Veröffentlichungstag und Bekanntmachung des Hinweises auf die Patenterteilung: **05.12.2018**
(21) Anmeldenummer: 12740480.4
(22) Anmeldetag: 03.07.2012
(51) Int. Cl.: G01N 33/34, G01N 33/36, G01N 21/86, G01N 21/89

(54) **PRÜFGERÄT UND VERFAHREN ZUR KALIBRIERUNG EINES PRÜFGERÄTS**
TEST INSTRUMENT AND METHOD FOR CALIBRATING A TEST INSTRUMENT
APPAREIL DE CONTRÔLE ET PROCÉDÉ POUR ÉTALONNER UN APPAREIL DE CONTRÔLE

(30) Priorität: 04.07.2011 DE 102011106523
(43) Veröffentlichungstag der Anmeldung: 14.05.2014
(73) Patentinhaber: Giesecke+Devrient Currency Technology GmbH, 81677 München (DE)
(72) Erfinder: BLOSS, Michael, 81241 München (DE); DECKENBACH, Wolfgang, 83135 Schechen (DE); HEIMANN, Werner, 80807 München (DE); EHRL, Hans-Peter, 82515 Wolfratshausen (DE); KERST, Erich, 85570 Unterföhring (DE)
(86) Internationale Anmeldenummer: PCT/EP2012/002796
(87) Internationale Veröffentlichungsnummer: WO 2013/004373

(56) Entgegenhaltungen:
- EP-A1- 0 195 168
- DE-A1- 4 439 972
- DE-A1-102007 038 754
- US-A- 5 991 046
- US-A1- 2010 231 897

## Beschreibung

Die Erfindung betrifft ein Prüfgerät zur Prüfung einer Materialbahn sowie ein Verfahren Kalibrierung des Prüfgeräts.

Bei der Herstellung einer Materialbahn, wie z.B. einer Faserstoffbahn, wird die Materialbahn mit Hilfe eines Transportsystems durch die verschiedenen Produktionsabschnitte der Materialbahn transportiert. Zur Prüfung der Materialbahn während ihrer Herstellung werden Prüfgeräte eingesetzt, die entlang eines Transportwegs der Materialbahn installiert sind. Während die

Materialbahn mit Hilfe eines Transportsystems an dem ortsfest installierten Prüfgerät vorbeitransportiert wird, detektiert das Prüfgerät Messwerte der Materialbahn, um eine oder mehrere bestimmte Eigenschaften der Materialbahn zu prüfen.

Zur Kalibrierung eines Prüfgeräts, das entlang des Transportwegs einer Materialbahn angeordnet ist und zur Prüfung einer Materialbahn bei deren Herstellung eingesetzt wird, wird üblicherweise ein Kalibriermedium in die Messebene des Prüfgeräts eingebracht, um mit dem Prüfgerät einen Kalibrier-Messwert des Kalibriermediums zu detektieren. Das Kalibriermedium wird zu diesem Zweck - während einer Unterbrechung der Materialbahn-Prüfung - manuell an das Prüfgerät angelegt, so dass temporär, an Stelle der Materialbahn, das Kalibriermedium in die Messebene des Prüfgeräts eingebracht ist. Dem Kalibriermedium ist ein bestimmter Sollwert zugeordnet, den das Prüfgerät im Idealfall bei einer Messung des Kalibriermediums detektiert. Beim Kalibrieren wird die Abweichung des tatsächlichen Messwerts von diesem Sollwert festgestellt. Nach erfolgter Kalibrierung wird das Prüfgerät so eingestellt, dass der Messwert des Prüfgeräts dem zu dem Kalibriermedium gehörigen Sollwert entspricht. DE 4439972 und US 2010/0231897 offenbaren verwandte Prüfgeräte und Verfahren zur Kalibrierung eines Prüfgerätes zur Prüfung einer Materialbahn.

Das Kalibriermedium weist üblicherweise einen bestimmten Messabschnitt auf, in dem der Kalibrier-Messwert detektiert wird. Der Sollwert, den das Prüfgerät innerhalb des Messabschnitts detektieren soll, ist üblicherweise außerhalb des Messabschnitts auf das Kalibriermedium aufgedruckt. Die Person, die die Kalibrierung durchführt, liest diesen Sollwert ab und gibt ihn manuell in das Prüfgerät ein.

Nachteilig ist bei diesem Kalibrierverfahren, dass der Kalibrier-Messwert, den das Prüfgerät detektiert, nicht genau definiert und kaum reproduzierbar ist. Denn der Detektionsort innerhalb des Messabschnitts des Kalibriermediums, an dem das Prüfgerät den Kalibrier-Messwert detektiert, ist aufgrund des manuellen Einlegens und der mechanischen Toleranzen des Kalibriermediums, Schwankungen in allen drei Raumrichtungen unterworfen. Außerdem birgt das manuelle Einbringen des Kalibriermediums das Risiko einer fehlerhaften Kalibrierung aufgrund eines ungenauen oder falschen Einlegens des Kalibriermediums durch die Person, die das Kalibriermedium an das Prüfgerät anlegt.

Eine Aufgabe der vorliegenden Erfindung ist es, ein Prüfgerät und ein Kalibrierverfahren für das Prüfgerät anzugeben, das eine genauere Kalibrierung erlaubt.

Diese Aufgabe wird durch die Gegenstände der unabhängigen Ansprüche gelöst. In davon abhängigen Ansprüchen sind vorteilhafte Weiterbildungen und Ausgestaltungen der Erfindung angegeben.

Das Prüfgerät ist zur Prüfung einer Materialbahn ausgebildet und weist dazu einen entsprechenden Betriebsmodus auf, in dem das Prüfgerät eine Materialbahn auf bestimmte Eigenschaften prüfen kann. Zum Prüfen der Materialbahn detektiert das Prüfgerät mehrere Messwerte der an dem Prüfgerät vorbeitransportierten Materialbahn. Je nach Anwendung werden beispielsweise optische, elektrische, mechanische oder magnetische Eigenschaften der Materialbahn geprüft, z.B. um eine Kontrolle eines Produktionsschritts der Materialbahn durchzuführen. Das Prüfgerät kann aber auch nach der Herstellung, z.B. für eine Qualitätsprüfung der Materialbahn eingesetzt werden. Außerdem weist das Prüfgerät einen weiteren Betriebsmodus auf, in dem eine Kalibrierung des Prüfgeräts durchgeführt werden kann. In dem zum Kalibrieren des Prüfgeräts eingerichteten Betriebsmodus können Kalibrier-Messwerte dadurch aufgenommen werden, dass das Prüfgerät Messwerte eines Kalibriermediums detektiert, das dem Prüfgerät zugeordnet ist. Dazu werden eine Vielzahl von Kalibrier-Messwerten detektiert, wenn das Kalibriermedium zur Kalibrierung an dem Prüfgerät vorbeitransportiert wird. Die Betriebsmodi sind z.B. in einer Steuereinrichtung des Prüfgeräts einprogrammiert.

Das Prüfgerät weist ein Gehäuse auf, in dem Messelemente aufgenommen sind, die sowohl zur Detektion von Messwerten der Materialbahn als auch zur Detektion von Kalibrier-Messwerten des zum Kalibrieren des Prüfgerät vorgesehen Kalibriermediums ausgebildet sind. In dem Gehäuse des Prüfgeräts sind außerdem Antriebsmittel angeordnet, die dazu ausgebildet sind, das Kalibriermedium, das zum berührungslosen Vorbeitransportieren an dem Prüfgerät vorgesehen ist, durch eine berührungslose magnetische Wechselwirkung mit dem Kalibriermedium, an dem Prüfgerät vorbeizutransportieren.

Während bei der bisherigen Kalibrierung das Kalibriermedium statisch in die Messebene des Prüfgerät eingebracht wurde und daher nur ein einziger Kalibrier-Messwert des Kalibriermediums detektierbar war, wird das Kalibriermedium erfindungsgemäß zur Kalibrierung an dem Prüfgerät vorbeitransportiert. Durch das Vorbeitransportieren wird erreicht, dass beim Kalibrieren nicht nur einer, sondern eine Vielzahl von Kalibrier-Messwerten des Kalibriermediums an einer Vielzahl von Positionen entlang des Kalibriermediums detektiert werden können. Aufgrund der Vielzahl von Kalibrier-Messwerten kann eine größere Genauigkeit der Kalibrierung erzielt werden als es mit einem einzigen Kalibrier-Messwert möglich ist, der unvermeidlichen Schwankungen unterworfen ist. Aus der Vielzahl von Kalibrier-Messwerten kann ein resultierender Kalibrier-Messwert ermittelt werden, der nur geringen Schwankungen unterworfen ist. Beispielsweise kann durch Bildung des Mittelwerts der Vielzahl von Kalibrier-Messwerten die Schwankungen einzelner Kalibrier-Messwerte eliminiert werden. Dadurch wird eine genauere Kalibrierung des Prüfgeräts möglich.

Das Kalibriermedium wird jedoch nicht durch das Transportsystem an dem Prüfgerät vorbeitransportiert wird, das zum Vorbeitransportieren der Materialbahn verwendet wird, sondern das erfindungsgemäße Prüfgerät stellt durch seine Antriebsmittel eine unabhängige Transportmöglichkeit für das Kalibriermedium bereit. Das Kalibriermedium wird durch die Antriebsmittel des Prüfgeräts an dem Prüfgerät vorbeitransportiert. Die Antriebsmittel des Prüfgeräts werden jedoch nicht einfach durch zusätzliche Komponenten bereit gestellt, etwa durch Transportkomponenten, die außerhalb des Prüfgeräts und unabhängig von dem Prüfgerät angeordnet sind, sondern die Antriebsmittel werden in das Prüfgerät selbst integriert. Würden zusätzliche Transportkomponenten für das Kalibriermedium verwendet, müssten diese relativ zu dem Prüfgerät installiert und dazu justiert werden, um eine ausreichend genaue Kalibrierung des Prüfgeräts durchführen zu können. Durch die Integration der Antriebsmittel in das Prüfgerät wird erreicht, dass die Kalibrierung einfacher durchgeführt werden kann, da das Justieren etwaiger zusätzlicher Transportkomponenten relativ zu dem Prüfgerät entfällt.

Die Antriebsmittel zum Vorbei transportieren des Kalibriermediums sind also nicht außerhalb des Prüfgeräts angeordnet, etwa durch Befestigung an dem Gehäuse des Prüfgeräts, sondern die Antriebsmittel sind in dem Gehäuse des Prüfgeräts enthalten. Dadurch wird erreicht, dass die Antriebsmittel geschützt sind vor Einwirkungen aus der Umgebung, in der das Prüfgerät zur Prüfung der Materialbahn eingesetzt werden soll. Da die Antriebsmittel im selben Gehäuse wie die Messelemente des Prüfgeräts angeordnet sind, wird ein kompakter Aufbau des Prüfgeräts erreicht. Das Gehäuse des Prüfgeräts ist nichtmagnetisch.

In der Produktionsumgebung einer Materialbahn können äußere Einwirkungen auftreten, z.B. durch Feuchtigkeit oder Verunreinigung, die die Funktion der Antriebsmittel beeinträchtigen könnten. Durch ein Einkapseln der Antriebsmittel in das Gehäuse des Prüfgeräts werden die Antriebsmittel vor Feuchtigkeit oder Verunreinigungen aus dieser Umgebung geschützt, wie z.B. vor Verschmutzungen, Staub, Flüssigkeit, Wasser, deren Kontakt mit den Antriebsmitteln vermieden werden soll. Das Gehäuse des Prüfgeräts ist dazu z.B. spritzwasserdicht abgedichtet, um das ein Eindringen von Flüssigkeiten aus der Umgebung in das Gehäuse zu vermeiden.

Um trotz deren Anordnung der Antriebsmittel in dem Gehäuse ein Vorbeitransportieren des Kalibriermediums an dem Prüfgerät zu erreichen, werden Antriebsmittel verwendet, die für eine berührungslose Wechselwirkung mit dem Kalibriermedium ausgebildet sind und das Kalibriermedium berührungslos an dem Prüfgerät vorbeitransportieren können. Dazu wird eine magnetische Wechselwirkung zwischen dem Antriebsmittel und dem Kalibriermedium eingesetzt. Die zum Vorbeitransportieren des Kalibriermediums benötigte Antriebskraft wird durch eine berührungslose magnetische Wechselwirkung des Kalibriermediums mit den Antriebsmitteln des Prüfgeräts erzeugt. Die Antriebsmittel sind dazu innerhalb des Gehäuses des Prüfgeräts unmittelbar benachbart zu derjenigen Seite des Prüfgeräts angeordnet, die der vorbeizutransportierenden Materialbahn bzw. dem vorbeizutransportierenden Kalibriermedium zugewandt ist.

Die in dem Gehäuse des Prüfgeräts angeordneten Antriebsmittel weisen z.B. mehrere Magnete auf. Diese Magnete können Permanentmagnete oder auch Elektromagnete sein. Die Magnete sind so angeordnet, dass ein Kalibriermedium, das ebenfalls magnetische Elemente aufweist, mit Hilfe der Magnete der Antriebsmittel an dem Prüfgerät vorbeitransportiert werden kann. Das Vorbeitransportieren wird durch eine magnetische Wechselwirkung zwischen den Magneten der Antriebsmittel, die innerhalb des Gehäuses des Prüfgeräts angeordnet sind, und den magnetischen Elementen des (außerhalb des Prüfgeräts angeordneten) Kalibriermediums erreicht. Die Antriebsmittel werden zum Vorbeitransportieren des Kalibriermediums bewegt. Durch die Bewegung der Antriebsmittel werden die Magnete der Antriebsmittel derart bewegt, dass ein Kalibriermedium, welches magnetische Elemente aufweist und in einen Erfassungsbereich der Antriebsmittel eingebracht ist, durch die Wechselwirkung mit den bewegten Magneten der Antriebsmittel erfassbar ist und an dem Prüfgerät vorbeitransportierbar ist. Die Anordnung der Magnete der Antriebsmittel ist dabei derart auf die Anordnung der magnetischen Elemente auf dem Kalibriermedium abgestimmt, dass das Kalibriermedium, wenn die Magnete der Antriebsmittel bewegt werden, synchron mit einer Bewegung der Magnete an dem Prüfgerät vorbeitransportierbar ist. Alternativ ist es auch möglich, die Antriebsmittel nicht zu bewegen sondern statische Antriebsmittel zu verwenden, z.B. positionsfeste Elektromagnete, die innerhalb des Gehäuses des Prüfgeräts entlang der Transportrichtung des Kalibriermediums angeordnet sind und die phasenversetzt mit Strom angesteuert werden, um das Kalibriermedium durch magnetische Wechselwirkung an dem Prüfgerät vorbeizutransportieren.

Durch die Anordnung der Magnete der Antriebsmittel in dem Gehäuse des Prüfgeräts wird erreicht, dass das Kalibriermedium räumlich definiert an dem Prüfgerät vorbeitransportierbar ist und dass die laterale Position des Kalibriermediums reproduzierbar ist. Somit sind reproduzierbare Detektionsorte auf dem Kalibriermedium und eine reproduzierbare Kalibrierung gewährleistet.

Die Magnete der Antriebsmittel und die magnetischen Elemente des Kalibriermediums sind bevorzugt so aufeinander abgestimmt, dass während des Vorbeitransportierens möglichst durchgehend, zumindest aber während des Detektierens der Messwerte des Kalibriermediums, eine attraktive Wechselwirkung zwischen den Magneten der Antriebsmittel und den magnetischen Elementen des Kalibriermediums erzeugt wird. Die Magnete der Antriebsmittel sind so in dem Prüfgerät angeordnet und in ihrer Magnetfeldstärke so gewählt, dass beim Detektieren der Messwerte des Kalibriermediums eine durchgehende laterale Führung des Kalibriermediums erreicht wird. Die attraktive Wechselwirkung führt aber auch zu einer Anziehung des Kalibriermediums an das Prüfgerät. Um dieser Anziehung entgegenzuwirken kann mindestens ein Führungselemente vorgesehen sein, das eine Gegenkraft auf das Kalibriermedium ausübt, so dass das Kalibriermedium in einen festen Abstand zum Prüfgerät daran vorbeitransportiert wird. Das Führungselement wird z.B. durch ein nichtmagnetisches Leitblech gebildet, das als Abstandshalter auf der Seite des Prüfgeräts angeordnet ist, die dem vorbeizutransportierenden Kalibriermedium zugewandt ist, so dass es zwischen dem Prüfgerät und dem vorbeitransportierten Kalibriermedium liegt. Das Leitblech kann zu diesem Zweck an dieser Seite des Gehäuses des Prüfgeräts befestigt sein.

In einem bevorzugten Ausführungsbeispiel weist das Prüfgerät mindestens zwei der Antriebsmittel auf, die jeweils für eine berührungslose Wechselwirkung mit dem Kalibriermedium ausgebildet sind und die quer zur Transportrichtung des Kalibriermediums zueinander versetzt angeordnet sind. Außerdem sind diese Antriebsmittel vorzugsweise so ausgebildet und angeordnet, dass sie synchron zueinander mit dem Kalibriermedium wechselwirken können. Zum Beispiel sind die Magnete dieser Antriebsmittel synchron zueinander bewegbar. Insbesondere können die zueinander versetzten Antriebsmittel parallel zueinander angeordnet und gleichartig ausgebildet sein. Beispielsweise sind dazu zwei oder mehrere Antriebsräder auf derselben Achse montiert und deren Phasenlage so gewählt, dass deren Magnete deckungsgleich zueinander montiert sind. Durch die Verwendung mindestens zweier quer zur Transportrichtung versetzter Antriebsmittel wird erreicht, dass die Transportlage und Transportrichtung des Kalibriermediums sehr gut definiert ist, eine vollkommen geradlinige Bewegung des Kalibriermediums gewährleistet ist. Ferner wird dadurch die laterale Stabilität des Vorbeitransportierens erhöht, was insbesondere den Transportbeginn des in den Erfassungsbereich der Antriebsmittel eingebrachten Kalibriermediums erleichtert.

Die Magnete der Antriebsmittel sind insbesondere so angeordnet, dass die magnetischen Pole der einander benachbarten Magnete abwechselnd entgegengesetzt zueinander orientiert sind, so dass durch die Bewegung der Magnete der Antriebsmittel, in dem Erfassungsbereich der Antriebsmittel abwechselnd die Kraft eines magnetische Nordpols und eines magnetischen Südpols bereitgestellt werden kann. Im Fall von zwei oder mehreren parallelen zueinander laufenden Antriebsmittel wird auf diese Weise ein Vorbeitransportieren des Kalibriermediums in schräger Lage des Kalibriermediums vermieden, falls das Kalibriermedium, das auch mit abwechselnd gepolten Magneten ausgestattet ist, versehentlich schräg in den Erfassungsbereich der Antriebsmittel eingebracht wurde. Bei gleicher Polung der einander benachbarten Magnete (sowohl bei Kalibriermedium als auch bei Antriebsmittel) kann es dagegen zu einem schrägen Vorbeitransportieren kommen, wenn das Kalibriermedium so schräg eingelegt wird, dass es von Magneten der beiden Antriebsmitteln erfasst wird, die entlang der Transportrichtung zueinander versetzt sind.

Die Antriebsmittel weisen beispielsweise mindestens ein Antriebsrad auf, wobei die Magnete der Antriebsmittel durch eine Rotation des Antriebsrads um seine Rotationsachse bewegbar sind. Die Magnete können dazu direkt auf dem Antriebsrad befestigt sein oder auf einem Trägerelement, auf dem die Magnete befestigt sind und das durch die Rotation des Antriebsrads bewegt ist. Das Trägerelement ist z.B. ein um das Antriebsrad umlaufender Riemen, insbesondere Zahnriemen, oder eine um das Antriebsrad umlaufende Kette.

Die Antriebsmittel, insbesondere das eine oder mehrere Antriebsrad, weisen bevorzugt einen magnetischen oder magnetisierbaren Körper auf, an dem die Magnete der Antriebsmittel befestigt sind. Dadurch wird der magnetische Fluss zwischen mindestens zwei der Magnete des Antriebsmittel, deren magnetische Pole radial entgegengesetzt zueinander orientiert sind, vergrößert. Im Vergleich zu einzelnem Magneten bzw. zu Befestigung der Magnete an einem nichtmagnetischen Körper ist dadurch eine größere magnetische Kraft der Magnete erreicht, so dass die magnetische Wechselwirkung über eine größere Distanz möglich ist. Damit wird auch bei größerem Abstand zwischen dem Antriebsmittel und dem Kalibriermedium ein sicheres und definiertes Vorbeitransportieren des Kalibriermediums ermöglicht. Der magnetische oder magnetisierbare Körper wird z.B. durch ein magnetisches/magnetisierbares Antriebsrad oder ein magnetisches/ magnetisierbares Trägerelement, insbesondere einen magnetischen/ magnetisierbaren Riemen oder eine magnetische/ magnetisierbare Kette, gebildet.

In einigen Ausführungsbeispielen wird mindestens ein Antriebsrad verwendet, entlang dessen Umfang die Magnete derart angeordnet sind, dass die Magnetfeldlinien des jeweiligen Magneten bezüglich der Rotationsachse des Antriebsrads radial nach außen weisen. Zum Beispiel weist jeweils dazu genau einer der magnetischen Pole des jeweiligen Magneten bezüglich einer Rotationsachse des Antriebsrads radial nach außen. Vorzugsweise sind entlang des Umfangs des Antriebsrads mehrere Magnete angeordnet, deren magnetischer Nordpol radial nach außen weist und mehrere Magnete, deren magnetischer Südpol radial nach außen weist. Dabei sind entlang des Umfangs des Antriebsrads benachbarte Magnete vorzugsweise derart angeordnet, dass abwechselnd der magnetische Nordpol bzw. der magnetische Südpol radial nach außen orientiert ist. Alternativ können auch alle Magnete eines Antriebsrads mit ihren Nordpolen radial nach außen orientiert sein oder alle mit ihren Südpolen radial nach außen. Um durchgehend eine anziehende Wechselwirkung zu erhalten, würden die Magnete des Kalibriermediums im ersten Fall so angeordnet, dass alle magnetischen Südpole zu dem Antriebsrad weisen, im zweien Fall so, dass alle magnetischen Nordpole zu dem Antriebsrad weisen.

Beispielsweise ist die Rotationsachse des mindestens einen Antriebsrads parallel zur Transportebene des Kalibriermediums orientiert, in welcher das Kalibriermedium zum Kalibrieren an dem Prüfgerät vorbeitransportiert wird, und senkrecht zur Transportrichtung des Kalibriermediums orientiert. Die Rotationsachse des mindestens einen Antriebsrads kann aber auch senkrecht zu der Transportebene des Kalibriermediums orientiert sein. In anderen Ausführungsbeispielen weist das Antriebsmittel mindestens zwei Antriebsräder und ein Trägerelemerit auf, auf dem die Magnete befestigt sind, wobei die Magnete der Antriebsmittel an der den Antriebsrädern abgewandten Seite des Trägerelements entlang des Trägerelements angeordnet sind.

Die Erfindung betrifft auch eine Anordnung aus zwei Prüfgeräten, die in Bezug auf den Transportweg der Materialbahn bzw. des Kalibriermediums einander gegenüberliegen. Vorzugsweise ist aber nur eines der gegenüberliegende Prüfgeräte mit einem erfindungsgemäßen Antriebsmittel ausgestattet und das diesem gegenüberliegende Prüfgerät nicht. Im Vergleich zu einer Ausstattung beider Prüfgeräte mit Antriebsmitteln für das Kalibriermedium ist dies vorteilhaft, denn es ist dann nicht nötig, mehrere Antriebsmittel aufeinander abzustimmen, z.B. was deren Phasenlage der Magnete betrifft. Außerdem lässt sich dadurch vermeiden, dass das Kalibriermedium versehentlich in umgekehrter Lage eingebracht wird.

Zum Kalibrieren wird das Prüfgerät von der Materialbahn (temporär) entfernt und das Prüfgerät in den Kalibrier-Betriebsmodus versetzt. In diesem Betriebsmodus wird die Bewegung der Antriebsmittel des Prüfgeräts gestartet. Anschließend wird das Kalibriermedium (z.B. manuell) an der zur Aufnahme von Messwerten vorgesehenen Seite des Prüfgeräts angeordnet und dort in den Erfassungsbereich der Antriebsmittel gebracht, in dem die berührungslose Wechselwirkung der Antriebsmittel erfolgen kann, z.B. deren magnetische Kraft zur Verfügung steht. Durch die berührungslose Wechselwirkung mit den Antriebsmitteln des Prüfgeräts wird das Kalibriermedium anschließend an dem Prüfgerät entlang der Transportrichtung vorbeitransportiert, insbesondere durch eine berührungslose magnetische Wechselwirkung. Während des Vorbeitransportierens des Kalibriermediums detektiert das Prüfgerät mit Hilfe seiner Messelemente eine Vielzahl von Kalibrier-Messwerten an verschiedenen Positionen innerhalb eines Messabschnitts des Kalibriermediums. Die Vielzahl der detektierten Messwerte wird anschließend zum Kalibrieren des Prüfgeräts verwendet.

Vorzugsweise wird das Kalibriermedium in den Erfassungsbereich der Antriebsmittel erst eingebracht, nachdem die Bewegung der Antriebsmittel gestartet wurde. Damit wird das manuelle Einbringen des Kalibriermediums in den Erfassungsbereich der Antriebsmittel erleichtert im Vergleich zu einem Einbringen des Kalibriermediums bei ruhendem Antriebsmittel. Denn im Fall einer abwechselnden entgegengesetzten Polung der Magnete der Antriebsmittel könnte es bei ruhendem Antriebsmittel eine Abstoßungsstellung auftreten, in der die Magnete der Antriebsmittel und die des einzubringenden Kalibriermediums einander abstoßen. Bei bewegtem Antriebsmittel wechseln dagegen die Abstoßungsstellung und Anziehungsstellung aufgrund der abwechselnden Magnetpolung einander ab. Beim manuellen Einbringen des Kalibriermediums wird daher auf alle Fälle eine Stellung der Magnete der Antriebsmittel erreicht, die eine anziehende Kraft auf das Kalibriermedium ausübt. Ferner wird damit ein ergonomisch günstigeres Einbringen des Kalibriermediums ermöglicht. Denn wenn die Kraft der bewegten Antriebsmittel auf das Kalibriermedium bereits beim manuellen Einbringen des Kalibriermediums wirkt, kann die Bedienperson diese beim Einbringen des Kalibriermediums wahrnehmen und erhält damit eine unmittelbare Rückmeldung dafür, dass das Kalibriermedium den Erfassungsbereichs der Antriebsmittel erreicht hat.

Das Kalibriermedium ist dazu ausgebildet ist, dass es durch eine berührungslose Wechselwirkung mit den Antriebsmitteln, insbesondere durch die Bewegung der Antriebsmittel, an dem Prüfgerät vorbeitransportierbar ist. Dazu weist das Kalibriermedium magnetische Elemente auf, die derart auf die Magnete der Antriebsmittel abgestimmt sind, dass das Kalibriermedium durch eine magnetische Wechselwirkung mit den Antriebsmitteln an dem Prüfgerät vorbeitransportiert werden kann. Das Kalibriermedium kann so, wenn die Magnete der Antriebsmittel bewegt werden, synchron mit einer Bewegung der Magnete der Antriebsmittel an dem Prüfgerät vorbeitransportiert werden.
Um ein falsches Einlegen des Kalibriermediums zu vermeiden ist das Kalibriermedium bevorzugt nur einseitig mit magnetischen Elementen versehen. Daher kann auch das Kalibriermedium nur in der richtigen Lage von den Antriebsmitteln erfasst und transportiert werden. Die magnetischen Elemente des Kalibriermediums können Permanentmagnete oder magnetisierbare Bestandteile des Kalibriermediums sein, z.B. einer oder mehrere ferromagnetische Bereiche.
Das Kalibriermedium weist vorzugsweise einen Messabschnitt auf, in dem, während des Vorbeitransportierens des Kalibriermediums an dem Prüfgerät, eine Vielzahl von Messwerten detektiert wird. Der Messabschnitt ist so ausgebildet, dass aus den Messwerten dieses Messabschnitts sowohl eine Vielzahl von Kalibrier-Messwerten ermittelt werden kann als auch mindestens der zum Kalibrieren des Prüfgeräts benötigter Sollwert. Damit wird erreicht, dass die Messelemente des Prüfgeräts gleichzeitig die Vielzahl der Kalibrier-Messwerte detektieren, aus denen ein resultierender Messwert des Kalibriermediums ermittelt wird, und den Sollwert, den das Prüfgerät im Idealfall detektiert und auf den das Prüfgerät nach dem Kalibrieren eingestellt wird. Die bisher notwendige manuelle Eingabe des von dem Kalibriermedium abgelesenen Sollwerts ist daher nicht mehr notwendig. Dass der Messabschnitt beide Informationen, den Sollwert und die Kalibrier-Messwerte, liefert ist vorteilhaft gegenüber einem bisherigen Kalibriermedium, bei dem einfach ein konventioneller optischer Barcode getrennt von der Kalibrierprobe auf das Kalibriermedium aufgebracht wird. Denn durch die Messelemente des Prüfgeräts ist ein zusätzlicher, z.B. neben dem Messabschnitt aufgeklebter Barcode normalerweise nicht detektierbar, insbesondere bei nicht-optischer Prüfung oder bei mangelnder optischer Auflösung des Prüfgeräts. Zum Lesen eines zusätzlichen Barcode müsste daher eigens ein Barcode-Leser an/in dem Prüfgerät bereitgestellt werden. Dies ist bei dem Messabschnitt, der beides, den Sollwert und die Kalibrier-Messwerte liefert, nicht notwendig.

Beispielsweise ist in dem Messabschnitt des Kalibriermediums eine Kalibrierprobe und ein der Kalibrierprobe überlagerter Barcode vorhanden, der mindestens einen zur Kalibrierung des Prüfgeräts benötigten Sollwert repräsentiert, auf den das Prüfgerät eingestellt werden soll. Der Barcode ist derart über der Kalibrierprobe angeordnet, dass die Kalibrierprobe abschnittsweise durch Streifenelemente des Barcodes abgedeckt ist. Während des Vorbeitransportierens wird der Messabschnitt (schrittweise oder kontinuierlich) durch Detektieren der Messwerte abgetastet. Die in den Zwischenräumen zwischen den Streifenelementen des Barcodes detektierten Messwerte werden als Kalibrier-Messwerte verwendet und der zum Kalibrieren benötigte Sollwert wird aus der Abfolge der Streifenelemente des Barcodes ermittelt. Aus den Kalibrier-Messwerten wird mindestens ein resultierender Messwert des Prüfgeräts gebildet, z.B. durch Berechnung des Mittelwerts der Vielzahl von Messwerten. Der Barcode ist z.B. ein optischer Barcode, ein magnetischer Barcode oder ein Dicken-Barcode, je nach Messprinzip des Prüfgeräts.

Das Prüfgerät hat vorzugsweise auch einen Betriebsmodus, in dem das Prüfgerät - quasi offline - als Laborgerät benutzt wird, um auch außerhalb der Produktionsumgebung der Materialbahn, Materialproben der Materialbahn detektieren zu können. Das Prüfgerät ermöglicht durch seine Antriebsmittel, dass - an Stelle des Kalibriermediums - eine Materialprobe geprüft werden kann, sofern diese an einem dafür ausgebildeten Probenträger befestigt wird. Der Probenträger ist dazu analog zu dem oben beschriebenen Kalibriermedium ausgebildet und z.B. mit entsprechenden Magneten ausgestattet.

Um das Prüfgerät zur Prüfung der Materialbahnprobe einzusetzen, werden folgende Schritte durchgeführt:
- Befestigen der Materialbahnprobe in einem Messabschnitt eines Probenträgers,
- Anordnen des Probenträgers an einer zur Aufnahme von Messwerten vorgesehenen Seite des Prüfgeräts derart, dass der Probenträger den Erfassungsbereich der (ggf. zuvor in Bewegung gesetzten) Antriebsmittel des Prüfgeräts erreicht,
- Vorbeitransportieren des Probenträgers an dem Prüfgerät entlang der Transportrichtung mit Hilfe der Antriebsmittel,
- Detektieren einer Vielzahl von Messwerten an verschiedenen Positionen innerhalb eines Messabschnitts der Materialbahnprobe mit Hilfe der Messelemente des Prüfgeräts, während der Probenträger an dem Prüfgerät vorbeitransportiert wird,
- Verwenden der detektierten Messwerte zum Prüfen der Materialbahnprobe.

Nachfolgend wird die Erfindung beispielhaft anhand der folgenden Figuren erläutert. Es zeigen:
- Figur 1: Eine Anordnung aus zwei gegenüberliegenden Prüfgeräten zur Prüfung einer Materialbahn,
- Figur 2: eine Kalibrierprobe (Fig. 2a), ein Kalibriermedium (Fig. 2b) und zwei der Magnete, mit denen das Kalibriermedium ausgestattet ist (Fig. 2c),
- Figur 3: Seitenansicht zweier gegenüberliegender Prüfgeräte und dazwischen hindurchtransportiertes Kalibriermedium (Fig. 3a) und Draufsicht auf das untere Prüfgerät (Fig. 3b),
- Figur 4: erstes Ausführungsbeispiel für Antriebsmittel zum Vorbeitransportieren des Kalibriermediums,
- Figur 5: zweites Ausführungsbeispiel für Antriebsmittel zum Vorbeitransportieren des Kalibriermediums,
- Figur 6: drittes Ausführungsbeispiel für Antriebsmittel zum Vorbeitransportieren des Kalibriermediums.

Während des Vorbeitransportierens der Materialbahn an dem erfindungsgemäßen Prüfgerät detektiert das Prüfgerät Messwerte der Materialbahn, um aus diesen Messwerten auf Eigenschaften der Materialbahn zu schließen. Die Eigenschaften können z.B. optische Remission, Transmission, Lumineszenz sein oder magnetische Eigenschaften, z.B. eines magnetischen Druckbilds oder eines Sicherheitsfadens, oder mechanische Eigenschaften, z.B. die Dicke der Materialbahn oder deren Oberflächenbeschaffenheit, etc.. Insbesondere ist das erfindungsgemäße Prüfgerät dazu ausgebildet, lumineszierende Eigenschaften einer Materialbahn zu überprüfen, bei deren Herstellung lumineszierende Substanzen auf die Materialbahn aufgebracht oder in die Materialbahn eingebracht werden. Beispielsweise wird das Prüfgerät zum Prüfen einer Papierbahn in einer Papiermaschine verwendet und ist dazu derart innerhalb der Papiermaschine angeordnet ist, dass es zum Prüfen von optischen Eigenschaften der Papierbahn während der Herstellung der Papierbahn einsetzbar ist, wenn die Papierbahn durch die Papiermaschine transportiert wird. Zum Kalibrieren wird das Prüfgerät, das normalerweise entlang eines Transportwegs der Materialbahn installiert ist, üblicherweise temporär aus der Messposition zur Prüfung der Materialbahn entfernt und in dieser entfernten Position kalibriert. Alternativ kann das Prüfgerät während der Kalibrierung in derjenigen Position entlang des Transportwegs der Materialbahn angeordnet sein, in der das Prüfgerät auch die Materialbahn prüft, z.B. wenn Produktion der Materialbahn unterbrochen ist.

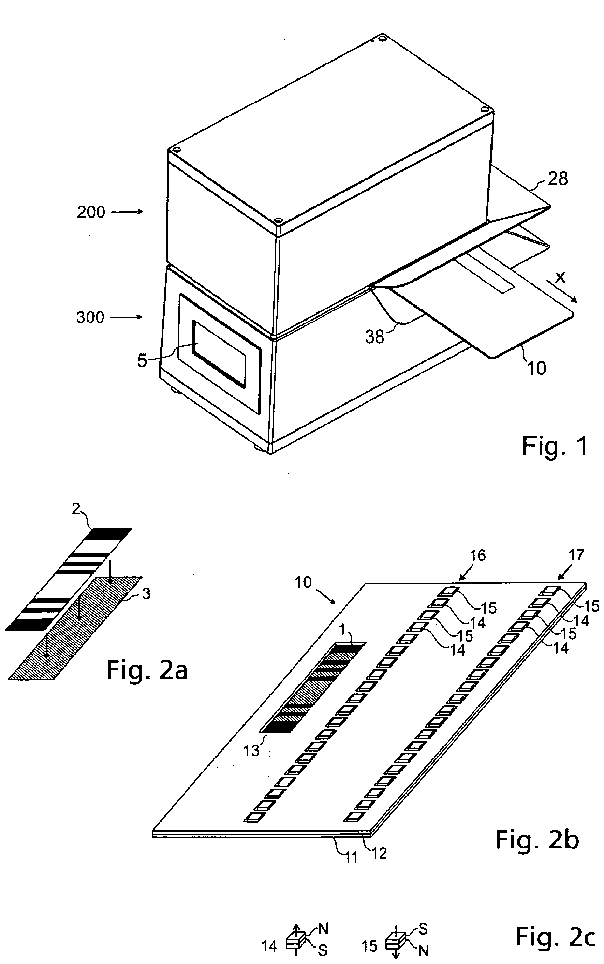

Figur 1 zeigt eine Anordnung aus zwei einander gegenüberliegenden Prüfgeräten 200, 300 zur Prüfung einer Materialbahn, zwischen denen ein Kalibriermedium 10 entlang der Transportrichtung x hindurch transportiert wird, um dieses beidseitig abzutasten. Zur Prüfung einer Materialbahn wird, an Stelle des Kalibriermediums 10, die Materialbahn zwischen den beiden Prüfgeräten 200, 300 hindurchtransportiert. An ihrer Frontseite sind die beiden Prüfgeräte mit Leitblechen 28, 38 ausgestattet, welche die Materialbahn bzw. das Kalibriermedium 10 in ihrer Transportebene T zwischen den Prüfgeräten hindurch führen. Die beiden Prüfgeräte 200, 300 sind elektronisch miteinander verbunden (nicht gezeigt), um Steuerbefehle oder Daten auszutauschen. Das Prüfgerät 300 weist ein Display 5 zur Ausgabe der Ergebnisse der Materialbahnprüfung auf. An Stelle der Anordnung aus Figur 1 ist es genauso möglich, nur das Prüfgerät 300 zu verwenden, z.B. für eine einseitige Prüfung der Materialbahn.

Figur 2b zeigt eine Detailansicht eines Kalibriermediums 10, welches mit einer Kalibrierprobe 3 ausgestattet ist, der ein Barcode 2 überlagert ist. Der Barcode 2 ist z.B. auf einer transparenten Folie aufgebracht und wird auf eine Kalibrierprobe 3 aufgeklebt, um deren feste Zuordnung zueinander zu gewährleisten, vgl. Figur 2a. Die mit dem Barcode 2 ausgestattete Kalibrierprobe 3 wird zwischen die beiden Platten 11, 12 des Kalibriermediums so eingeklemmt, dass sie durch eine Aussparung 13 der oberen Platte 12 sichtbar ist. In weitere Aussparungen der oberen Platte 12 sind eine Vielzahl von Magneten 14, 15 eingelegt. Die Magnete werden durch die magnetische Wechselwirkung mit der unteren Platte 11 festgehalten, die zu diesem Zweck magnetisch ist. Die obere Platte 12 ist nichtmagnetisch. Alternativ können die Magnete 14, 15 natürlich auch anders befestigt sein, z.B. durch Kleben oder Klemmen. Die Magnete 14 und 15 liegen in zwei parallelen Reihen 16, 17 vor, innerhalb derer sich jeweils ein Magnet 14 und ein Magnet 15 abwechseln. Die Magnete 14 sind mit ihrem magnetischen Nordpol nach oben angeordnet, die Magnete 15 dagegen umgekehrt, mit ihrem magnetischen Südpol nach oben, vgl. Figur 2c.

In den transparenten Abschnitten des Barcodes 2 (Barcode-Zwischenräume), in denen die Kalibrierprobe 3 sichtbar ist, detektiert das Prüfgerät beim Abtasten der Kalibrierprobe 3 eine Vielzahl von Kalibrier-Messwerten. Die Kalibrier-Messwerte sind z.B. optische Messwerte der Kalibrierprobe, die zum Kalibrieren des Prüfgeräts verwendet werden. Beim Abtasten des Kalibriermediums sorgt der Barcode für eine Modulation der durch das Prüfgerät detektierten Messwerte, da die Barcode-Streifen lichtabsorbierend sind. Diese Modulation wird durch das Prüfgerät dekodiert, um einen oder mehrere für die Kalibrierung benötigte Sollwerte zu ermitteln, die der Kalibrierprobe 3 zugeordnet sind.

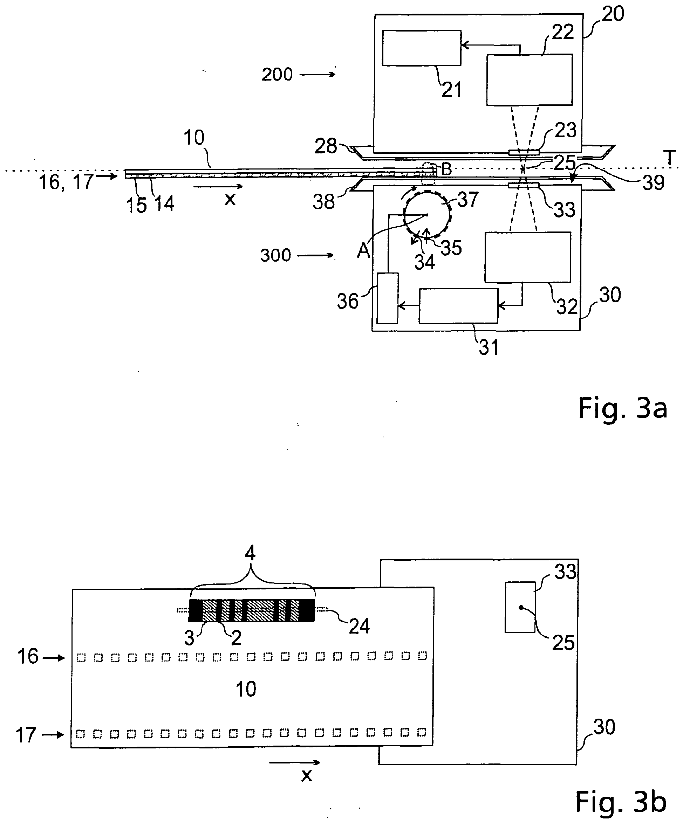

In Figur 3a sind die beiden Prüfgeräte 200, 300 in Seitenansicht dargestellt, wobei für die Darstellung jeweils die dem Betrachter zugewandte Seitenwand der Gehäuse 20, 30 in Figur 3a weggelassen ist. In diesem Beispiel ist nur das Prüfgerät 300 ein erfindungsgemäßes Prüfgerät. Das Prüfgerät 200 ist vorteilhaft, um eine doppelseitige Abtastung der Materialbahn durchführen zu können, aber in Bezug auf die Erfindung nicht notwendig. In dem Prüfgerät 200 kann eine Steuereinrichtung 21 vorgesehen sein, die das Detektieren von Messwerten durch die Messelemente 22 steuert. Um eine versehentliches falsches Einlegen des Kalibriermediums 10 zu unterbinden, weist das Prüfgerät 200 jedoch keine erfindungsgemäßen Antriebsmittel auf. Denn das Kalibriermedium 10 kann dann nur in der gezeigten Orientierung durch die Antriebsmittel des Prüfgeräts 300 erfasst werden, wenn die (gestrichelt einzeichneten) Magnete 14, 15 nach unten weisen, vgl. Figur 3a. Das Prüfgerät 200 detektiert mit Hilfe seiner (nur schematisch gezeigten) Messelemente 22 Messwerte von der Oberseite der Kalibrierprobe 3 des Kalibriermediums 10. Die Kalibrierprobe 3 kann an ihrer Oberseite mit einem weiteren Barcode ausgestattet sein, der sich von dem Barcode 2 auf deren Unterseite, der von dem Prüfgerät 300 detektiert wird, im Allgemeinen unterscheidet, um dem Prüfgerät 200 ebenfalls einen Sollwert für dessen Kalibrierung zuzuführen. Der weitere Barcode wird z.B. durch eine weitere Folie bereitgestellt, die auf die Oberseite der Kalibrierprobe aufgeklebt ist.

Das Prüfgerät 300 weist ein Gehäuse 30 auf, in dem sowohl die (nur schematisch gezeigten) Messelemente 32 als auch die Antriebsmittel 34, 35, 37 zum Transportieren des Kalibriermediums 10: eingekapselt sind. Das Gehäuse 30 ist vollständig abgeschlossen, um Verschmutzung und Feuchtigkeit aus der Umgebung von den Antriebsmitteln und von den Messelementen 32 des Prüfgeräts 300 fernzuhalten. Eine Steuereinrichtung 31 steuert die Messelemente 32 und verarbeitet die detektierten Messwerte. In der Steuereinrichtung 31 sind Betriebsmodi zum Detektieren der Materialbahn um zum Kalibrieren des Prüfgeräts 300 (und ggf. des Prüfgeräts 200) eingerichtet. Die Messelemente 32 sind in diesem Beispiel zur Prüfung von optischen Eigenschaften der Materialbahn ausgebildet und detektieren die Messwerte der Materialbahn bzw. des Kalibriermediums an dem Detektionsort 25 durch ein Fenster 33 hindurch, das auf der der Transportebene T zugewandten Seite 39 des Gehäuses 30 vorhanden ist. Im Fall optischer Messelemente 32 umfassen diese z.B. zumindest eine Lichtquelle und einen Detektor. Figur 3b zeigt eine Draufsicht auf das erfindungsgemäße Prüfgerät 300 aus Figur 3a. Durch Bewegung des Kalibriermediums 10 entlang der Transportrichtung x wird der Messabschnitt 24 des Kalibriermediums durch den Detektionsort 25 bewegt, wobei die Messelemente 32 eine Vielzahl von Messwerten des Messabschnitts 24 detektieren.

Die Steuereinrichtung 31 steuert außerdem den Motor 36, durch den das Antriebsrad 37 in Rotation um die Rotationsachse A versetzt werden kann. Durch die Rotation des Antriebsrads 37 werden die Magnete 34, 35 bewegt, die einander abwechselnd entlang des Umfangs des Antriebsrads 37 angeordnet sind. Die Magnete 34 sind so angeordnet, dass ihr magnetischer Nordpol radial nach außen weist, die Magnete 35 dagegen so, dass ihr magnetischer Südpol radial nach außen weist. Durch die Rotation des Antriebsrads 37 wirkt in dem Erfassungsbereich B der Antriebsmittel abwechselnd die magnetische Kraft der Magnete 34 und der Magnete 35, vgl. Figur 3a.

Zum Kalibrieren wird zuerst die Rotation des Antriebsrads 37 gestartet und anschließend das Kalibriermedium 10 zwischen die beiden Prüfgeräte 200, 300 eingeschoben, bis einer von dessen Magnete 14,15 in den Erfassungsbereich B der Magnete 34, 35 des Antriebsrads gelangt. Sobald durch die Rotation des Antriebsrads 37 einer der Magnete 34 des Antriebsrads in unmittelbare Nähe der Gehäuseseite 39 gelangt, wirkt in dem Erfassungsbereich B dessen magnetische Kraft anziehend auf den ersten Magneten 15 des eingeschobenen Kalibriermediums 10 (in Figur 3a gezeigte Stellung). Durch die anziehende magnetische Wechselwirkung zwischen den Magneten 15 und 34 wird das Kalibriermedium 10 entlang der Transportrichtung x synchron mit der Rotation des Antriebsrads 37 bewegt. Die Vorschubkraft, die das Kalibriermedium an dem Prüfgerät 300 vorbeitransportiert, wird abwechselnd durch die anziehende magnetische Wechselwirkung zwischen den Magneten 14 und 35 und die anziehende magnetische Wechselwirkung zwischen den Magneten 15 und 34 erzeugt. Das Leitelement 38 wirkt dabei als Abstandshalter für das Kalibriermedium 10, damit das Kalibriermedium 10 sich nicht auf die Gehäuseseite 39 zu bewegt. Sobald der letzte Magnet des Kalibriermediums den Erfassungsbereich B passiert hat, wirkt keine Vorschubkraft mehr auf das Kalibriermedium 10.

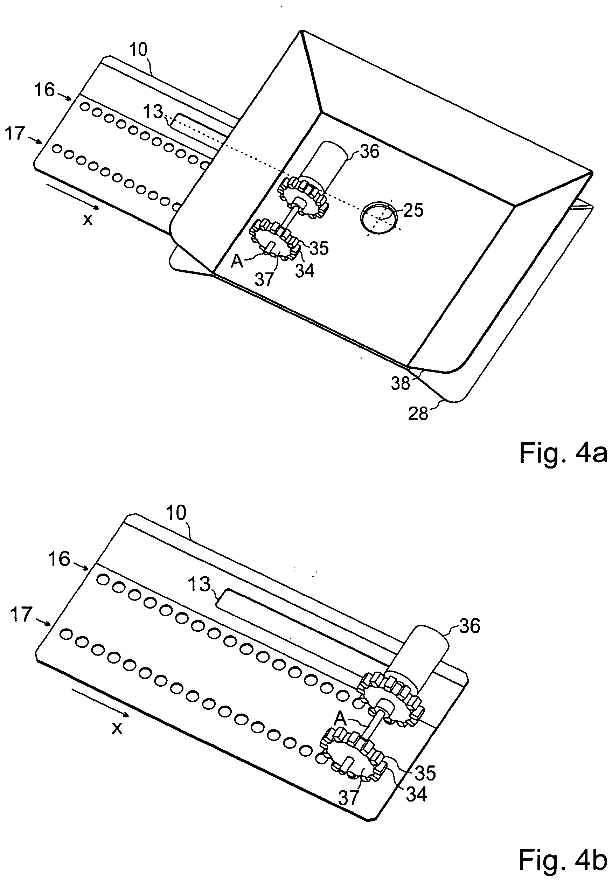

In Figur 4a ist eine erste Ausführungsform der Antriebsmittel gezeigt, wie sie für das Beispiel der Figuren 3a und 3b verwendbar sind. Die Antriebsmittel 34, 35, 37 und der Motor 36 sind alle in dem Gehäuse des Prüfgeräts angeordnet, das Kalibriermedium 10 außerhalb. In der Darstellung sind das Gehäuse des Prüfgeräts und die restlichen Bestandteile des Prüfgeräts jedoch weggelassen und nur die Leitelemente 28, 38 gezeigt. Das Leitelement 38 weist eine Öffnung auf, durch welche die Messelemente des Prüfgeräts auf den Detektionsort 25 gerichtet sind. Als Antriebsmittel werden in diesem Beispiel zwei quer zur Transportrichtung versetzte Antriebsräder 37 verwendet, die auf derselben Rotationsachse A befestigt sind und durch den Motor 36 angetrieben werden. Entlang deren Umfang sind die Magnete 34, 35 - wie oben beschrieben - mit abwechselnder magnetischer Polung angeordnet. Die Magnete 34, 35 der beiden Antriebsräder 37 sind mit ihrer magnetische Polung entlang des Umfangs deckungsgleich zueinander angeordnet. Bei dem Kalibriermedium 10 sind die Positionen der Magnete 14, 15 entlang der beiden Reihen 16, 17 in den Figuren 4a, 4b nur schematisch gezeigt und die in der Aussparung 13 anzuordnende Kalibrierprobe weggelassen. Figur 4b zeigt die Anordnung aus Figur 4a ohne die Leitelemente 38, 28.

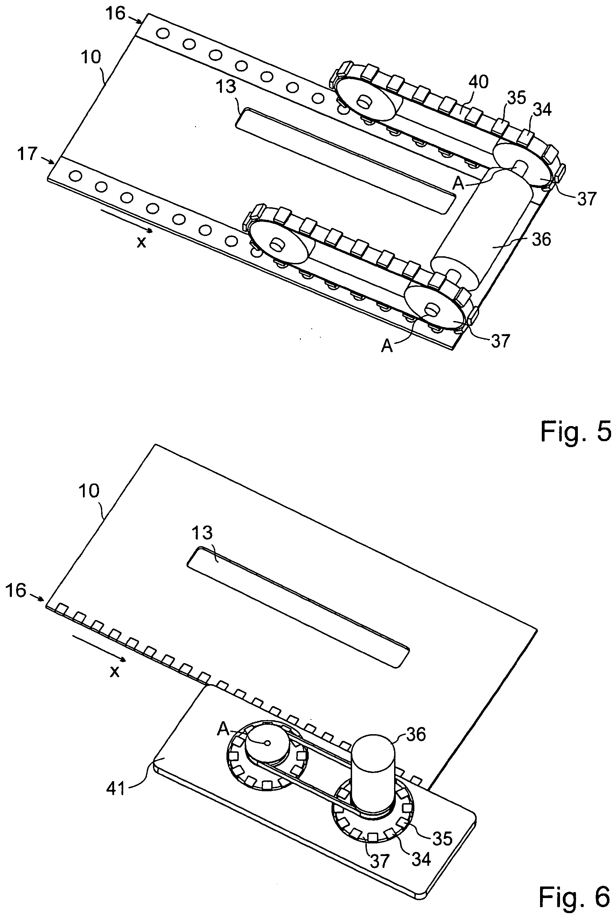

Figur 5 zeigt ein zweites Ausführungsbeispiel der erfindungsgemäßen Antriebsmittel. Die Antriebsmittel 34, 35, 37, 40 und der Motor 36 sind alle in dem Gehäuse des Prüfgeräts angeordnet, das Kalibriermedium 10 außerhalb. Der Motor 36 treibt über seine Rotationsachse A zwei Antriebsräder 37 an, die quer zur Transportrichtung x zueinander versetzt sind. Beide Antriebsräder sind jeweils über einen Riemen 40 mit einem weiteren, entlang der Transportrichtung versetzten Antriebsrad verbunden, um auch dieses anzutreiben. Auf den beiden Riemen 40 sind jeweils Magnete 34, 35 mit abwechselnder magnetischer Polung angeordnet. Das Kalibriermedium 10 wird bei diesem Ausführungsbeispiel zu Beginn des Transports nur durch die Wechselwirkung mit zwei Magneten entlang der Transportrichtung x bewegt, im Laufe des Vorbeitransportierens aber mit allen Magneten, die auf der dem Kalibriermedium 10 zugewandten Seite des Riemens angeordnet sind.

In Figur 6 ist ein drittes Ausführungsbeispiel der erfindungsgemäßen Antriebsmittel dargestellt. Die Antriebsmittel 34, 35, 37 und der Motor 36 sind alle in dem Gehäuse des Prüfgeräts angeordnet, das Kalibriermedium 10 außerhalb. Der Motor 36 treibt das Antriebsrad 37 an, das durch Kopplung mittels eines Riemens ein zweites Antriebsrad mit antreibt. Wie im Beispiel der Figuren 3 und 4 sind auch bei diesen Antriebsrädern 37 entlang des Umfangs abwechselnd Magnete 34, 35 angeordnet, deren magnetische Polung abwechselnd radial nach außen bzw. radial nach innen weist. Im Unterschied zu den vorherigen Beispielen werden hier jedoch Antriebsräder 37 verwendet, deren Rotationsachse A senkrecht zur Transportebene T des Kalibriermediums 10 orientiert ist. Die beiden Antriebsräder sind in entsprechende Vertiefungen einer Platte 41 eingesetzt, die innerhalb des Gehäuses des Prüfgeräts angeordnet ist oder einen Teil der Gehäusewand bildet. An Stelle der beiden in Figur 6 gezeigten Antriebsräder kann auch ein einziges Antriebsrad 37 verwendet werden. In derselben Orientierung, d.h. mit der Rotationsachse A senkrecht zur Transportebene T, können analog auch die Antriebsmittel aus Figur 4 oder die aus Figur 5 verwendet werden. Bei dieser Orientierung der Rotationsachsen A wird, anders als bei dem Kalibriermedium der Figuren 2b und 3b, eine Kante des Kalibriermediums 10 mit einer Reihe 16 von Magneten 14, 15 ausgestattet, vgl. Figur 6. Damit können diese mit den Magneten 34, 35 der Antriebsräder 37 eine magnetische Wechselwirkung ausreichender Stärke erzeugen.

## Patentansprüche

1. Prüfgerät (300) zur Prüfung einer entlang einer Transportrichtung (x) an dem Prüfgerät vorbeitransportierbaren Materialbahn, wobei das Prüfgerät folgendes aufweist:
- Messelemente (32), die zum Detektieren von Messwerten der an dem Prüfgerät (300) vorbeitransportierbaren Materialbahn sowie zum Detektieren von Messwerten eines an dem Prüfgerät entlang der Transportrichtung (x) vorbeitransportierbaren Kalibriermediums (10) ausgebildet sind,
- einen Betriebsmodus, in dem das Prüfgerät mit Hilfe des Kalibriermediums (10), das zum Kalibrieren entlang der Transportrichtung (x) an dem Prüfgerät vorbeitransportierbar ist, kalibriert werden kann,
- eines oder mehrere Antriebsmittel (34, 35, 37), die dazu ausgebildet sind, das Kalibriermedium (10) durch eine berührungslose magnetische Wechselwirkung an dem Prüfgerät (30) vorbeizutransportieren,
- ein Gehäuse (30), in dem sowohl die Antriebsmittel (34, 35, 37) zum Vorbeitransportieren des Kalibriermediums (10) als auch die Messelemente (32) zum Detektieren der Messwerte des Kalibriermediums angeordnet sind.

2. Prüfgerät nach Anspruch 1, **dadurch gekennzeichnet, dass** die Antriebsmittel (34, 35, 37) durch das Gehäuse (30) des Prüfgeräts derart eingekapselt sind, dass die Antriebsmittel vor Feuchtigkeit und Verunreinigungen aus der Umgebung, in der das Prüfgerät (300) eingesetzt werden soll, geschützt sind.

3. Prüfgerät nach einem der vorhergehenden Ansprüche, **dadurch gekennzeichnet, dass** das Prüfgerät (30) mindestens jeweils zwei der Antriebsmittel (34, 35, 37) aufweist, die quer zur Transportrichtung (x) des Kalibriermediums (10) zueinander versetzt angeordnet sind und vorzugsweise so ausgebildet und angeordnet sind, dass sie synchron zueinander mit dem Kalibriermedium (10) wechselwirken können.

4. Prüfgerät nach einem der vorhergehenden Ansprüche, **dadurch gekennzeichnet, dass** die in dem Gehäuse (30) des Prüfgeräts angeordneten Antriebsmittel mehrere Magnete (34, 35) aufweisen, durch die ein Kalibriermedium (10), das magnetische Elemente (14, 15) aufweist, entlang der Transportrichtung (x) an dem Prüfgerät (300) vorbeitransportierbar ist, wobei das Vorbeitransportieren des Kalibriermediums durch eine magnetische Wechselwirkung zwischen den Magneten (34, 35) der Antriebsmittel und den magnetischen Elementen (14, 15) des Kalibriermediums (10) erreicht wird.

5. Prüfgerät nach Anspruch 4, **dadurch gekennzeichnet, dass** die Magnete (34, 35) der Antriebsmittel derart in dem Gehäuse des Prüfgeräts (300) angeordnet sind, dass die laterale Position des Kalibriermediums (10), die dieses beim Vorbeitransportieren des Kalibriermediums an dem Prüfgerät (300) einnimmt, reproduzierbar ist.

6. Prüfgerät nach einem der Ansprüche 4 bis 5, **dadurch gekennzeichnet, dass** die Magnete (34, 35) der Antriebsmittel derart in dem Gehäuse des Prüfgeräts (300) angeordnet und derart ausgebildet sind, dass, während des Vorbeitransportierens des Kalibriermediums (10) an dem Prüfgerät (300), eine attraktive Wechselwirkung zwischen den Magneten (34, 35) der Antriebsmittel und den magnetischen Elementen (14, 15) des Kalibriermediums erzeugt wird, um beim Detektieren der Messwerte des Kalibriermediums durchgehend eine laterale Führung des Kalibriermediums (10) zu erreichen.

7. Prüfgerät nach einem der Ansprüche 4 bis 6, **dadurch gekennzeichnet, dass** einander benachbarte Magnete (34, 35) der Antriebsmittel so angeordnet sind, dass die magnetischen Pole der einander benachbarten Magnete (34, 35) abwechselnd entgegengesetzt zueinander orientiert sind, so dass, durch die Bewegung die Magnete (34, 35) der Antriebsmittel, in dem Erfassungsbereich (B) der Antriebsmittel abwechselnd die Kraft eines magnetische Nordpols und eines magnetischen Südpols bereitgestellt werden kann.

8. Prüfgerät nach einem der Ansprüche 4 bis 7, **dadurch gekennzeichnet, dass** die Antriebsmittel mindestens ein Antriebsrad (37) aufweisen, entlang dessen Umfang die Magnete (34,35) derart angeordnet sind, dass die Magnetfeldlinien des jeweiligen Magneten (34, 35) bezüglich einer Rotationsachse (A) des Antriebsrads (37) radial nach außen weisen.

9. Prüfgerät nach Anspruch 8, **dadurch gekennzeichnet, dass** entlang des Umfangs des Antriebsrads (37) mehrere Magnete (34) angeordnet sind, deren magnetischer Nordpol radial nach außen weist und mehrere Magnete (35), deren magnetischer Südpol radial nach außen weist, wobei die entlang des Umfangs des Antriebsrads (38) aufeinanderfolgenden Magnete (34, 35) vorzugsweise derart angeordnet sind, dass bei den einander benachbarten Magneten (34, 35) abwechselnd der magnetische Nordpol bzw. der magnetische Südpol radial nach außen orientiert ist.

10. Prüfgerät nach einem der Ansprüche 8 bis 9, **dadurch gekennzeichnet, dass** die Antriebsmittel mindestens ein Antriebsrad (37) und ein Trägerelement (40) aufweisen, das zumindest abschnittweise entlang des Umfang des Antriebsrads (37) angeordnet ist und das durch die Rotation des Antriebsrads (37) bewegbar ist, wobei die Magnete (34, 35) an der dem Antriebsrad (37) abgewandten Seite auf dem Trägerelement (40) angeordnet sind.

11. Verfahren zum Kalibrieren eines Prüfgeräts, insbesondere des Prüfgeräts (300) nach einem der vorhergehenden Ansprüche, wobei das Prüfgerät zur Prüfung einer entlang einer Transportrichtung (x) an dem Prüfgerät vorbeitransportierbaren Materialbahn ausgebildet ist und ein Gehäuse (30) aufweist, in dem sowohl Antriebsmittel (34, 35, 37) zum Vorbeitransportieren eines Kalibriermediums (10) als auch Messelemente (32) zum Detektieren von Messwerten der Materialbahn und von Messwerten des Kalibriermediums angeordnet sind, wobei zum Kalibrieren des Prüfgeräts folgende Schritte durchgeführt werden:
- Anordnen eines Kalibriermediums (10) an einer zur Aufnahme von Messwerten vorgesehenen Seite (39) des Prüfgeräts derart, dass das Kalibriermedium (10) den Erfassungsbereich (B) der Antriebsmittel erreicht,
- Vorbeitransportieren des Kalibriermediums (10) an dem Prüfgerät entlang der Transportrichtung (x) mit Hilfe der Antriebsmittel (34, 35, 37), wobei das Kalibriermedium (10) durch eine berührungslose magnetische Wechselwirkung mit den Antriebsmitteln des Prüfgeräts an dem Prüfgerät vorbeitransportiert wird,
- Detektieren einer Vielzahl von Messwerten an verschiedenen Positionen innerhalb eines Messabschnitts (24) des Kalibriermediums (10) mit Hilfe der Messelemente (32) des Prüfgeräts, während das Kalibriermedium (10) an dem Prüfgerät vorbeitransportiert wird,
- Kalibrieren des Prüfgeräts mit Hilfe der detektierten Messwerte des Kalibriermediums (10).

12. Verfahren nach Anspruch 11, **dadurch gekennzeichnet, dass** das Kalibriermedium (10) in den Erfassungsbereich (B) der Antriebsmittel erst eingebracht wird, nachdem eine Bewegung der Antriebsmittel (34, 35, 37) gestartet wurde.

13. Verfahren nach einem der Ansprüche 11 bis 12, **dadurch gekennzeichnet, dass** das Kalibriermedium (10) mehrere magnetische Elemente (14, 15) aufweist, deren Anordnung auf dem Kalibriermedium (10) derart auf die Anordnung von Magneten (34, 35) der Antriebsmittel des Prüfgeräts abgestimmt ist, dass das Kalibriermedium (10), wenn die Magnete (34, 35) der Antriebsmittel bewegt werden, synchron mit einer Bewegung der Magnete (34, 35) der Antriebsmittel an dem Prüfgerät vorbeitransportiert wird.

14. Verfahren nach einem der Ansprüche 11 bis 13, **dadurch gekennzeichnet, dass** aus der Vielzahl von Messwerten, die von einem Messabschnitt (24) des Kalibriermediums während des Vorbeitransportierens des Kalibriermediums (10) detektiert werden, sowohl eine Vielzahl von Kalibrier-Messwerten ermittelt wird, als auch mindestens ein zur Kalibrierung des Prüfgeräts benötigter Sollwert ermittelt werden, wobei in dem Messabschnitt (24) des Kalibriermediums insbesondere eine Kalibrierprobe (3) und ein der Kalibrierprobe (3) überlagerter Barcode (2) vorhanden sind, der mindestens einen zur Kalibrierung des Prüfgeräts benötigten Sollwert repräsentiert.

15. Verfahren nach Anspruch 14, **dadurch gekennzeichnet dass** die Kalibrierprobe (3) auf ihrer dem Prüfgerät (300) zugewandten und ihrer dem Prüfgerät (300) abgewandten Seite jeweils einen Barcode aufweist, wobei der dem Prüfgerät (300) zugewandte Barcode (2) einen zur Kalibrierung des Prüfgeräts (300) benötigten Sollwert repräsentiert und der von dem Prüfgerät (300) abgewandte Barcode einen zur Kalibrierung eines weiteren Prüfgeräts (200) benötigten Sollwert repräsentiert, das zu dem Prüfgerät (300) gegenüberliegend angeordnet ist.

## Claims

1. A checking device (300) for checking a material web transportable past the checking device along a transport direction (x), wherein the checking device has the following:
- measurement elements (32) configured for detecting measurement values of the material web transportable past the checking device (300) and for detecting measurement values of a calibration medium (10) transportable past the checking device along the transport direction (x);
- an operating mode in which the checking device can be calibrated with the aid of the calibration medium (10) which is transportable past the checking device along the transport direction (x) for calibration;
- one or several drive means (34, 35, 37) configured for transporting the calibration medium (10) past the checking device (30) through a contactless magnetic interaction;
- a housing (30) having arranged therein both the drive means (34, 35, 37) for transporting the calibration medium (10) past and the measurement elements (32) for detecting the measurement values of the calibration medium.

2. The checking device according to claim 1, **characterized in that** the drive means (34, 35, 37) are encapsulated by the housing (30) of the checking device such that the drive means are protected from moisture and contaminants from the environment in which the checking device (300) is to be used.

3. The checking device according to any of the preceding claims, **characterized in that** the checking device (300) has at least respectively two of the drive means (34, 35, 37) arranged mutually offset transversely to the transport direction (x) of the calibration medium (10) and preferably configured and arranged to interact with the calibration medium (10) in mutual synchronism.

4. The checking device according to any of the preceding claims, **characterized in that** the drive means arranged in the housing (30) of the checking device have several magnets (34, 35) through which a calibration medium (10) having magnetic elements (14, 15) is transportable past the checking device (300) along the transport direction (x), wherein the transporting past of the calibration medium is obtained through a magnetic interaction between the magnets (34, 35) of the drive means and the magnetic elements (14, 15) of the calibration medium (10).

5. The checking device according to claim 4, **characterized in that** the magnets (34, 35) of the drive means are arranged in the housing of the checking device (300) such that the lateral position of the calibration medium (10) which the latter assumes upon the transporting of the calibration medium past the checking device (300) is reproducible.

6. The checking device according to any of claims 4 to 5, **characterized in that** the magnets (34, 35) of the drive means are arranged in the housing of the checking device (300) and configured such that, while the calibration medium (10) is being transported past the checking device (300), an attractive interaction between the magnets (34, 35) of the drive means and the magnetic elements (14, 15) of the calibration medium is produced in order to obtain a lateral guidance of the calibration medium (10) continuously upon the detecting of the measurement values of the calibration medium.

7. The checking device according to any of claims 4 to 6, **characterized in that** mutually adjacent magnets (34, 35) of the drive means are arranged with the magnetic poles of the mutually adjacent magnets (34, 35) alternately oriented opposite to each other, so that, through the motion of the magnets (34, 35) of the drive means, the force of a magnetic north pole and of a magnetic south pole can be alternately provided in the capture region (B) of the drive means.

8. The checking device according to any of claims 4 to 7, **characterized in that** the drive means have at least one drive wheel (37) along the circumference of which the magnets (34, 35) are arranged such that the magnetic field lines of the respective magnet (34, 35) point radially outward with reference to a rotational axis (A) of the drive wheel (37).

9. The checking device according to claim 8, **characterized in that** along the circumference of the drive wheel (37) there are arranged several magnets (34) the magnetic north poles of which point radially outward and several magnets (35) the magnetic south poles of which point radially outward, wherein the magnets (34, 35) succeeding each other along the circumference of the drive wheel (38) are preferably arranged such that the magnetic north poles and the magnetic south poles of the mutually adjacent magnets (34, 35) are alternately oriented radially outward.

10. The checking device according to any of claims 8 to 9, **characterized in that** the drive means have at least one drive wheel (37) and a carrier element (40) which is arranged at least in certain portions along the circumference of the drive wheel (37) and is movable through the rotation of the drive wheel (37), wherein the magnets (34, 35) are arranged on the carrier element (40) on the side facing away from the drive wheel (37).

11. A method for calibrating a checking device, in particular of the checking device (300) according to any of the preceding claims, wherein the checking device is configured for checking a material web transportable past the checking device along a transport direction (x) and has a housing (30) having arranged therein both drive means (34, 35, 37) for transporting a calibration medium (10) past and measurement elements (32) for detecting measurement values of the material web and measurement values of the calibration medium, wherein for calibrating the checking device the following steps are carried out:
- arranging a calibration medium (10) on a side (39) of the checking device provided for sensing measurement values such that the calibration medium (10) reaches the capture region (B) of the drive means;
- transporting the calibration medium (10) past the checking device along the transport direction (x) with the aid of the drive means (34, 35, 37), wherein the calibration medium (10) is transported past the checking device through a contactless magnetic interaction with the drive means of the checking device;
- detecting a multiplicity of measurement values at different positions within a measurement portion (24) of the calibration medium (10) with the aid of the measurement elements (32) of the checking device while the calibration medium (10) is being transported past the checking device;
- calibrating the checking device with the aid of the detected measurement values of the calibration medium (10).

12. The method according to claim 11, **characterized in that** the calibration medium (10) is only brought into the capture region (B) of the drive means after a motion of the drive means (34, 35, 37) has been started.

13. The method according to any of claims 11 to 12, **characterized in that** the calibration medium (10) has several magnetic elements (14, 15) the arrangement of which on the calibration medium (10) is coordinated with the arrangement of magnets (34, 35) of the drive means of the checking device such that the calibration medium (10), when the magnets (34, 35) of the drive means are moved, is transported past the checking device in synchronism with a motion of the magnets (34, 35) of the drive means.

14. The method according to any of claims 11 to 13, **characterized in that** from the multiplicity of measurement values that are detected by a measurement portion (24) of the calibration medium while the calibration medium (10) is being transported past, both a multiplicity of calibration measurement values are established and at least one target value required for calibrating the checking device is established, wherein in the measurement portion (24) of the calibration medium there are present in particular a calibration sample (3) and a bar code (2) superimposed on the calibration sample (3), said bar code representing at least one target value required for calibrating the checking device.

15. The method according to claim 14, **characterized in that** the calibration sample (3) respectively has a bar code on its side facing the checking device (300) and its side facing away from the checking device (300), wherein the bar code (2) facing the checking device (300) represents a target value required for calibrating the checking device (300) and the bar code facing away from the checking device (300) represents a target value required for calibrating a further checking device (200) which is arranged so as to oppose the checking device (300).

## Revendications

1. Appareil de contrôle (300) destiné au contrôle d'une bande de matière transportable le long d'une direction de transport (x) en passant par l'appareil de contrôle, l'appareil de contrôlé comportant ce qui suit :
- des éléments de mesure (32) conçus pour la détection de valeurs de mesure de la bande de matière transportable en passant par l'appareil de contrôle (300) ainsi que pour la détection de valeurs de mesure d'un moyen d'étalonnage (10) transportable en passant par l'appareil de contrôle le long de la direction de transport (x),
- un mode de fonctionnement dans lequel l'appareil de contrôle peut être étalonné à l'aide du moyen d'étalonnage (10) transportable le long de la direction de transport (x) en passant par l'appareil de contrôle aux fins de l'étalonnage,
- un ou plusieurs dispositifs d'entraînement (34, 35, 37) conçus pour, par une interaction magnétique sans contact, transporter le moyen d'étalonnage (10) en passant par l'appareil de contrôle (30),
- un boîtier (30) dans lequel sont agencés tant les dispositifs d'entraînement (34, 35, 37) pour le transport du moyen d'étalonnage (10) que les éléments de mesure (32) pour la détection des valeurs de mesure du moyen d'étalonnage.

2. Dispositif selon la revendication 1, **caractérisé en ce que** les dispositifs d'entraînement (34, 35, 37) sont encapsulés de telle manière par le boîtier (30) de l'appareil de contrôle que les dispositifs d'entraînement sont protégés de l'humidité et des impuretés de l'environnement dans lequel l'appareil de contrôle (300) est destiné à être utilisé.

3. Appareil de contrôle selon une des revendications précédentes, **caractérisé en ce que** l'appareil de contrôle (30) comporte au moins respectivement deux des dispositifs d'entraînement (34, 35, 37) qui sont agencés en quinconce l'un par rapport à l'autre transversalement à la direction de transport (x) du moyen d'étalonnage (10) et qui sont de préférence réalisés et agencés de telle manière qu'ils peuvent interagir avec le moyen d'étalonnage (10) en synchronisation l'un par rapport à l'autre.

4. Appareil de contrôle selon une des revendications précédentes, **caractérisé en ce que** les dispositifs d'entraînement agencés dans le boîtier (30) de l'appareil de contrôle comportent plusieurs aimants (34, 35) par lesquels un moyen d'étalonnage (10) comportant des éléments magnétiques (14, 15) est transportable le long de la direction de transport (x) en passant par l'appareil de contrôle (300), cependant que le transport du moyen d'étalonnage est obtenu par une interaction magnétique entre les aimants (34, 35) des dispositifs d'entraînement et les éléments magnétiques (14, 15) du moyen d'étalonnage (10).

5. Appareil de contrôle selon la revendication 4, **caractérisé en ce que** les aimants (34, 35) des dispositifs d'entraînement sont agencés de telle manière dans le boîtier de l'appareil de contrôle (300) que la position latérale du moyen d'étalonnage (10) que ce dernier adopte lors du transport du moyen d'étalonnage en passant par l'appareil de contrôle (300) est reproductible.

6. Appareil de contrôle selon une des revendications de 4 à 5, **caractérisé en ce que** les aimants (34, 35) des dispositifs d'entraînement sont agencés de telle manière dans le boîtier de l'appareil de contrôle (300) et sont réalisés de telle façon que, pendant le transport du moyen d'étalonnage (10) en passant par l'appareil de contrôle (300), une interaction attractive entre les aimants (34, 35) des dispositifs d'entraînement et les éléments magnétiques (14, 15) du moyen d'étalonnage est générée afin d'obtenir sans interruption un guidage latéral du moyen d'étalonnage (10) lors de la détection de valeurs de mesure du moyen d'étalonnage.

7. Appareil de contrôle selon une des revendications de 4 à 6, **caractérisé en ce que** des aimants (34, 35) voisins l'un de l'autre des dispositifs d'entraînement sont agencés de telle manière que les pôles magnétiques des aimants (34, 35) voisins l'un de l'autre sont orientés alternativement en opposition l'un par rapport à l'autre, de telle sorte que, par le mouvement des aimants (34, 35) des dispositifs d'entraînement, dans la zone de saisie (B) des dispositifs d'entraînement, en alternance la force d'un pôle magnétique nord et d'un pôle magnétique sud peut être mise à disposition.

8. Appareil de contrôle selon une des revendications de 4 à 7, **caractérisé en ce que** les dispositifs d'entraînement comportent au moins une roue d'entraînement (37) le long du périmètre de laquelle les aimants (34, 35) sont agencés de telle manière que les lignes de champ magnétique de l'aimant (34, 35) respectif s'étendent radialement vers l'extérieur par rapport à un axe de rotation (A) de la roue d'entraînement (37).

9. Appareil de contrôle selon la revendication 8, **caractérisé en ce que,** le long du périmètre de la roue d'entraînement (37), il y a plusieurs aimants (34) dont le pôle magnétique nord est tourné radialement vers l'extérieur et plusieurs aimants (35) dont le pôle magnétique sud est tourné radialement vers l'extérieur, cependant que les aimants (34, 35) se suivant le long du périmètre de la roue d'entraînement (38) sont de préférence agencés de telle manière que, aux aimants (34, 35) voisins l'un de l'autre, c'est alternativement le pôle magnétique nord et le pôle magnétique sud qui est orienté radialement vers l'extérieur.

10. Appareil de contrôle selon une des revendications de 8 à 9, **caractérisé en ce que** les dispositifs d'entraînement comportent au moins une roue d'entraînement (37) et un élément support (40) qui est, ou moins par sections, agencé le long du périmètre de la roue d'entraînement (37), et par lequel la rotation de la roue d'entraînement (37) peut être mise en mouvement, cependant que les aimants (34, 35) sont agencés sur l'élément support (40) sur le côté détourné de la roue d'entraînement (37).

11. Procédé d'étalonnage d'un appareil de contrôle, en particulier de l'appareil de contrôle (300) selon une des revendications précédentes, cependant que l'appareil de contrôle est conçu pour le contrôle d'une bande de matière transportable le long de la direction de transport (x) en passant par l'appareil de contrôle et comporte un boîtier (30) dans lequel sont agencés tant des dispositifs d'entraînement (34, 35, 37) pour le transport d'un moyen d'étalonnage (10) que des éléments de mesure (32) pour la détection des valeurs de mesure de la bande de matière et de valeurs de mesure du moyen d'étalonnage (10), cependant que, pour l'étalonnage de l'appareil de contrôle, les étapes suivantes sont effectuées :
- agencement du moyen d'étalonnage (10) sur un côté (39), prévu pour l'enregistrement de valeurs de mesure, de l'appareil de contrôle, de telle manière que le moyen d'étalonnage (10) atteint la zone de saisie (B) des dispositifs d'entraînement,
- transport du moyen d'étalonnage (10) en passant par l'appareil de contrôle le long de la direction de transport (x) à l'aide des dispositifs d'entraînement (34, 35, 37), cependant que le moyen d'étalonnage (10) est, par une interaction magnétique sans contact avec les dispositifs d'entraînement de l'appareil de contrôle, transporté en passant par l'appareil de contrôle,
- détection d'une pluralité de valeurs de mesure à différentes positions à l'intérieur d'une section de mesure (24) du moyen d'étalonnage (10) à l'aide des éléments de mesure (32) de l'appareil de contrôle pendant que le moyen d'étalonnage (10) est transporté en passant par l'appareil de contrôle,
- étalonnage de l'appareil de contrôle à l'aide des valeurs de mesure détectées du moyen d'étalonnage (10).

12. Procédé selon la revendication 11, **caractérisé en ce que** le moyen d'étalonnage (10) n'est introduit dans la zone de saisie (B) des dispositifs d'entraînement qu'après qu'un mouvement des dispositifs d'entraînement t (34, 35, 37) a été lancé.

13. Procédé selon une des revendications de 11 à 12, **caractérisé en ce que** le moyen d'étalonnage (10) comporte plusieurs éléments magnétiques (14, 15) dont l'agencement sur le moyen d'étalonnage (10) est accordé de telle manière avec l'agencement d' aimants (34, 35) des dispositifs d'entraînement de l'appareil de contrôle que le moyen d'étalonnage (10), quand les aimants (34, 35) des dispositifs d'entraînement sont mis en mouvement, est transporté en passant par l'appareil de contrôle en synchronisation avec un mouvement des aimants (34, 35) des dispositifs d'entraînement.

14. Procédé selon une des revendications de 11 à 13, **caractérisé en ce que,** parmi la pluralité de valeurs de mesure détectées par une section de mesure (24) du moyen d'étalonnage durant le transport du moyen d'étalonnage (10), non seulement une pluralité de valeurs de mesure d'étalonnage est déterminée, mais aussi au moins une valeur de consigne requise pour l'étalonnage de l'appareil de contrôle sont déterminées, cependant que, dans la section de mesure (24) du moyen d'étalonnage , il y a en particulier un échantillon d'étalonnage (3) et un code-barres (2) superposé à l'échantillon d'étalonnage (3) qui représente au moins une valeur de consigne requise pour l'étalonnage de l'appareil de contrôle.

15. Procédé selon la revendication 14, **caractérisé en ce que** l'échantillon d'étalonnage (3) comporte, sur son côté tourné vers l'appareil de contrôle (300) et sur son côté détourné de l'appareil de contrôle (300), respectivement un code-barres, cependant que le code-barres (2) tourné vers l'appareil de contrôle (300) représente une valeur de consigne requise pour l'étalonnage de l'appareil de contrôle (300), et que le code-barres détourné de l'appareil de contrôlé (300) représente une valeur de consigne requise pour l'étalonnage d'un autre appareil de contrôle (200) agencé en face de l'appareil de contrôle (300).
